# EUROPEAN PATENT SPECIFICATION

(11) **EP 4 034 054 B1**
(45) Date of publication and mention of the grant of the patent: **04.03.2026**
(21) Application number: 20867121.4
(22) Date of filing: 16.09.2020
(51) Int. Cl.: A61F 7/12, A61M 25/06, A61B 8/12, A61B 8/08, A61B 8/00, A61F 7/00, A61M 5/172, A61M 39/02

(54) **DEVICE TO COOL THE BRAIN AND TO DIAGNOSE AND TREAT GLIOBLASTOMA**
VORRICHTUNG ZUM KÜHLEN DES GEHIRNS UND ZUR DIAGNOSE UND BEHANDLUNG VON GLIOBLASTOMEN
DISPOSITIF DE REFROIDISSEMENT DU CERVEAU ET DE DIAGNOSTIC ET DE TRAITEMENT DU GLIOBLASTOME

(30) Priority: 25.09.2019 US 201962905996 P
(43) Date of publication of application: 03.08.2022
(73) Proprietor: Wong, Edward, Newport Beach, CA 92660 (US)
(72) Inventor: SOLEK, Roman, Pleasanton, CA 94556 (US); FIRTH, John, Nottingham, Nottinghamshire NG71EJ (GB); WONG, Edward, Newport Beach, CA 92660 (US)
(74) Representative: Arnold & Siedsma
(86) International application number: PCT/US2020/051084
(87) International publication number: WO 2021/061471

(56) References cited:
- EP-B1- 1 691 860
- US-A1- 2002 013 529
- US-A1- 2010 280 438
- US-A1- 2012 123 270
- US-A1- 2012 172 838
- US-A1- 2017 203 084
- EMONDS N., HASSLER W.E.: "New device to treat chronic subdural hematoma--hollow screw", NEUROLOGICAL RESEARCH, vol. 21, no. 1, 30 November 1998 (1998-11-30), GB , pages 77 - 78, XP009535331, ISSN: 0161-6412, DOI: 10.1080/01616412.1999.11740897

## Description

### Background

### Field of the Technology

The illustrated embodiments are directed to a unique approach to rapidly cool the brain to deep hypothermia (20 to 25 degrees Celsius using aCSF cooled to 1 - 10°C) safely and rapidly for the prevention of brain death in cases of lack of circulation to the brain, including such diseases as cardiac arrest due to myocardial infarction, stroke, and exsanguination.

### Description of the Prior Art

Fifty years ago, Professor Robert White demonstrated that cooling the brain of a monkey down to 15 degrees Celsius would protect the structure and function of the brain without blood circulating to the brain for one hour when tying all major vessels in the neck. When the ligatures around all the vessels of the neck were released after one hour, the monkey was given time to return to room temperature, after which testing showed no deterioration of memory, problem solving, and motor activity.

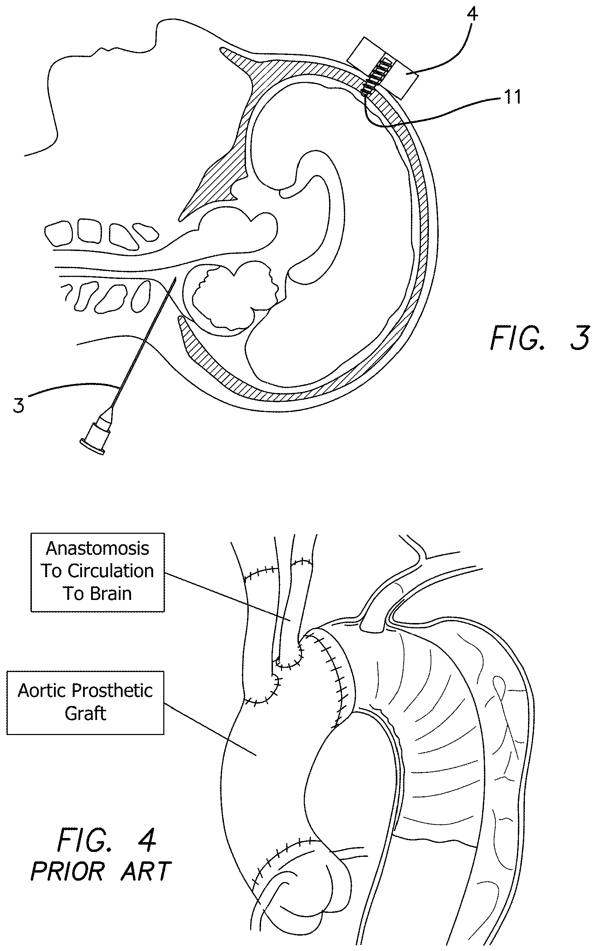

Professor White's novel work is currently being used successfully today by cardiothoracic surgeons who repair the aortic arch with an aortic prosthesis such as in cases of Marfan's syndrome with dissection of the aorta as depicted in Fig. 4. They can obtain 30 minutes of operating time with little to no blood to the brain if they pre-cool the brain to 10 degrees Celsius. By directing extremely cold saline into the subclavian artery, they can reach this temperature in the brain. This is quite successful resulting in prevention of brain damage with no loss of memory or function. Fig, 4 highlights the importance of knowing the broad medical literature outside of neurology and neurological surgery. In 1975 Griepp et al. first reported in the cardiothoracic literature that after lowering brain temperature to the range of deep hypothermia with cardiopulmonary bypass. They successfully preserved brain function despite cardiac arrest in their patients with replacement of the aortic arch. It is common medical practice for such surgery to include deep hypothermia to put the brain in "suspended animation" during the 30 minutes or longer of no blood to the brain.

The brain can be put into a state of suspended animation despite having no blood flow by lowering its temperature to extreme hypothermia. A 29-year-old radiologist, Anna Bågenholm, was reported in The Lancet (355 (9201) 375-376) to have been frozen in accidental submersion to 13.7 degrees Celsius with a flat electrocardiogram. Upon resuscitation 1 and return to normothermia, she had complete recovery of all mental faculties. Samuel Tisherman, MD, who has Department of Defense funding, was reported in November 2019 to treat a severely traumatized patient by cooling the brain to extreme hypothermia by using refrigerated saline into the cerebrovascular circulation. After two hours of hypothermia he was able to complete surgical repair and then resuscitate the patient.

Children who had fallen into a frozen lake have been reported to survive with no apparent brain damage after being submerged for one hour or longer in extreme hypothermia- Adults under similar challenging situations will not survive the freezing waters, for children may have an open fontanel as well as thinner skulls allowing for rapid cooling of the brain. Today, infants and children who are in danger of ischemic brain damage are treated with moderate to mild hypothermia with improved survival and less brain damage compared to children without lowering of their systemic temperature.

Many reports have studied the use of mild to moderate hypothermia, lowering the body temperature to only 33 degrees Celsius, in cases of heart attack with varying reports of success in outcome analysis. Lowering the body temperature to less than 33 degrees Celsius can result in cardiac arrhythmia due to deleterious effects on the ventricle, and bleeding problems due to interruption of platelet aggregation resulting in hemorrhage.

In cases where there is interruption of blood to the brain, we may have only minutes before there is irreversible brain damage. While cardiopulmonary resuscitation (CPR) has been the standard treatment for cardiac arrest, even successful cases may result in a stroke of brain tissues with significant disability. Besides cardiac arrest, massive stroke and exsanguination are major life-threatening problems that need to be treated within the first few minutes of lack of circulation to the brain. It is no wonder that the military is anxious to find a way to extend survival of the brain for one hour or longer with circulatory arrest. This search for a method to extend the "golden hour" will give the treating physician a chance to treat the immediate problem and its cause. If the brain can be put in suspended animation for one hour or longer, it will result in a paradigm shift in medical care for patients threatened with immediate brain death! Restoration of circulation and repair of injured organs and tissues will be possible if the brain can be protected from ischemic damage by utilizing deep hypothermia rapidly and effectively. Given one hour and not just five minutes time, the treating physician will have a chance to save the patient.

Document US 2012/123270 A1 describes an interventional system including a miniaturized device with ultrasound transducers and a processing means.

### Brief Summary

The invention is set out in the independent claims. Particular embodiments of the invention are set out in the dependent claims. Any subject matter contained herein that does not fall within the scope of the appended claims is considered as being useful for understanding the invention.

In the range of profound hypothermia (<14 degrees Celsius), such low temperatures may be achieved using the technique of cooling the brain with artificial cerebrospinal fluid (aCSF). Although subject to clinical refinement, for the purpose of this patent, a target temperature range of "deep" hypothermia is used consistent with the surgical technique of "deep hypotherntic circulatory arrest" (DHCA). A review of the history, current work, and rationale for this paradigm shift in approaching deep hypothermia of the brain is presented.

The illustrated embodiments of the present disclosure provide an apparatus and method for prevention of brain death due to interruption of intracranial circulation to the brain due to cardiac arrest, stroke, loss of blood due to exsanguination, and other causes. By selectively cooling the brain, it can be put into "suspended animation" for one hour or longer without circulating blood flow. Instead of only five minutes to save a patient with impending brain death, the treating physician will have one hour or longer to save the life of a patient. By cooling the cerebrospinal fluid directly, it is possible to cool the brain without directly entering the cerebrovascular circulation through cannulating the aorta or large vessels to the brain. The time it would take to cannulate the circulation makes it difficult to do rapidly, and the problems of systemic cooling may result in arrhythmias and hemorrhage.

Therefore, a paradigm shift in cooling the brain rapidly requires a new approach. The apparatus of the illustrated embodiments is necessary in order to rapidly enter the cisterna magna safely, accurately, and atraumatically. Since the first person available to insert a needle into the brain may be a paramedic, the procedure must be full-proof, easy to use, and rapidly administered. Once in place, the needle must be immobilized to a stationary anatomic site, and the sharp cutting tip of the needle must change its shape to prevent damage to brain tissue in the cisterna magna and the brainstem, In the case of battlefield situations, the design of a similar needle system for entry into a large vessel such as the femoral artery must be done safely and rapidly when used by a paramedic.

### Method for Cooling of the Brain through the Cisterna Magna

Safety, accuracy, and speed are the hallmarks of the illustrated embodiments to rapidly prevent brain death in emergencies dealing with patients who have acute interruption of circulation to the brain which will result in brain death. Clinically, the brain can be put in suspended animation during surgery to replace a dissecting aortic aneurysm by pumping refrigerated saline into the brain through a bypass into the subclavian artery. This is quite successfully done throughout the world in humans.

Our laboratory studies demonstrate that the brain in the experimental animal can be rapidly cooled to deep hypothermia or even lower to profound hypothermia. Cooling the cerebrospinal fluid (CSF) rapidly by circulating cooled artificial (aCSF) about the brain will also result in cooling the central nervous system (CNS) by targeting the blood vessels in the basal cisterns as well as the brain within the subarachnoid space. We have found that entry into the subarachnoid space can be accomplished rapidly and safely by entering the cisterna magna from the cranial-cervical junction posteriorly or from a lateral approach below the mastoid bone. Because of the proximity of the entering needle to the brainstem and to the vertebral arteries, this must be done under direct visualization using ultrasound direction *through* and about the needle. There must be a vent placed in the forehead (near the hair line) to evacuate aCSF out of the head and to allow for convection cooling. This vent is a new design for a trephine that is rapidly inserted, is stable, is safe from injury to the brain, is accurately placed, and is done by a single person semi-autonomously. There is a temperature differential with cooler fluid posteriorly near the occiput with the patient lying supine, while the exiting fluid is warmer near the frontal lobe. The subarachnoid space is relatively large in older patients, giving greater cooling effect with a larger volume of aCSF.

A different configuration allows for aCSF to exit the trephine and be recycled into the cisterna magna. The vent opening is connected to a sterile drainage system or to a pump system to recycle aCSF that is controlled via information from the tubing in the forehead for temperature and pressure measurement. Temperature of the exiting aCSF is monitored as well as the entering temperature at the cisterna magna. The cooling system is integrated with a refrigeration unit and a peristaltic pump. If a closed system is used, a filter system is in series with this closed system to remove debris and contaminants and infectious agents from the pathway from the front of the brain where the fluid is exiting; thereby, convection cooling will occur resulting in better cooling of the brain rapidly. A virtual "pump" created by convection cooling will act as an additional "motor" within the skull to facilitate rapid cooling.

In effect, the recirculating cooled aCSF is in a closed and sterile system that will cool the cisterna magna and structures of the brain within the subarachnoid space. Initial cooling is provided to the vessels at the base of the brain thence through the cooled blood circulating through the brain tissue. The cooled aCSF will directly cool the base of the brain and vital structures including memory. From there the cooled aCSF will pass around the brain within the subarachnoid space. In the supine position there is a differential in temperature between the cool fluid near the occiput and the warm fluid at the front of the brain where the fluid is exiting, thereby, convection cooling will occur resulting in better cooling of the brain rapidly. A virtual pump created by convection cooling will act as an additional pump within the skull to facilitate rapid cooling.

### Brief Description of the Drawings

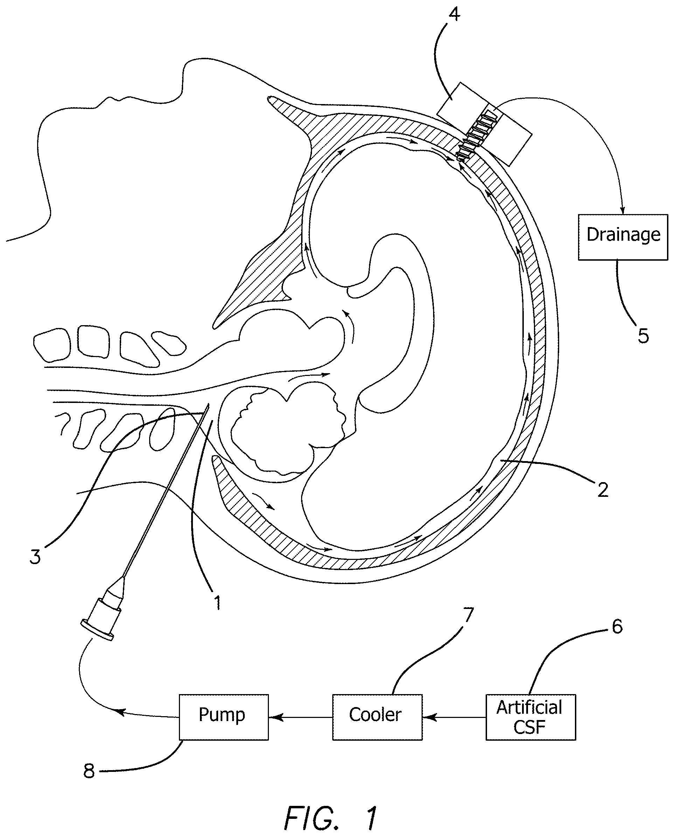

Fig. 1 is a simplified side cross sectional diagram of a human head showing the elements of the cooling system with a pump and cooler administering artificial CSF (aCSF) through a fixated needle in the cisterna magna, circulating the cooled aCSF through the basal cisterns, through the subarachnoid space about the cranium, and drainage to a sterile container.

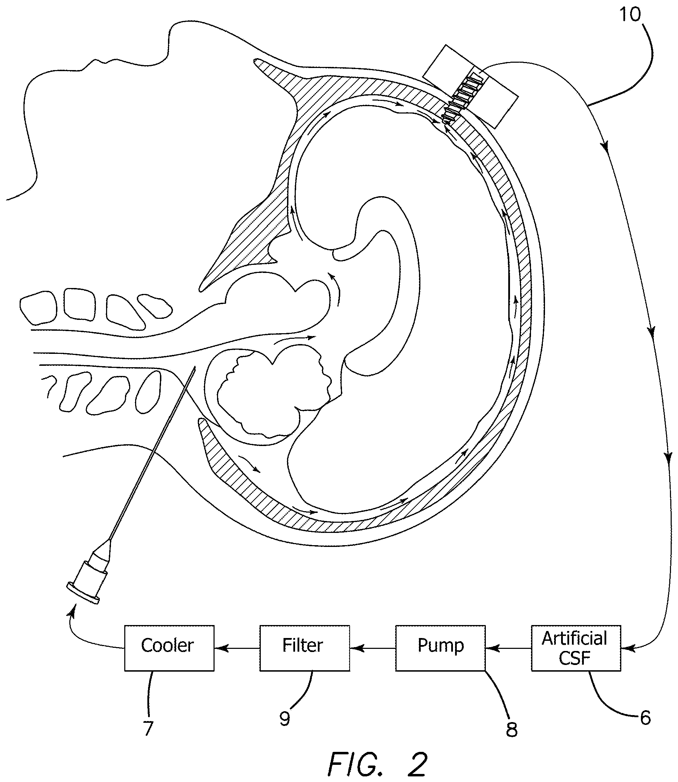

Fig. 2 is a simplified side cross sectional diagram of a human head showing recycling of aCSF from the exit port in the skull to a pump, filter, and cooling system, and then re-entering the subarachnoid space through the fixated needle in the cisterna magna.
Fig. 3 is a simplified side cross sectional diagram of a human head showing the specially designed unit, a semi-autonomous trephine, placed into the skull and into the subarachnoid space, for the purpose of exiting aCSF after it has circulated throughout the subarachnoid space. It also includes a temperature sensor and a pressure monitor.
Fig. 4 is a diagram of the prosthesis used in prior art surgical repair of a dissecting aortic aneurysm in Marfan's Syndrome with current technology that requires the use of deep hypothermia to prevent brain death, demonstrating that "suspended animation" by cooling the brain has been successfully used since 1975 throughout the world.

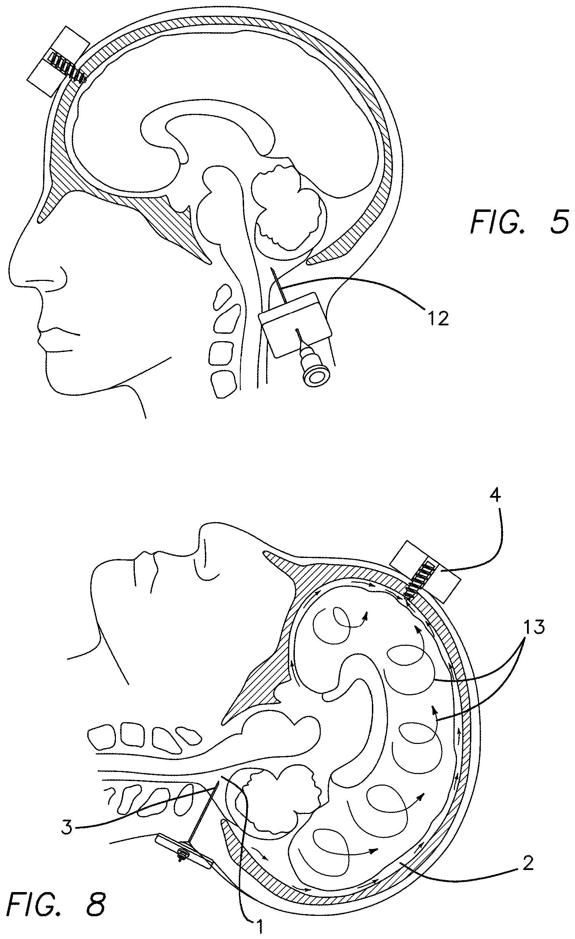

Fig. 5 is a simplified side cross sectional diagram of a human head that illustrates a new technique used to enter the cisterna magna from a lateral approach, which was first reported in 2018 using a different approach, which new technique has the advantage of being adjacent to the mastoid bone for locating and fixating the needle to a bony anatomical location,

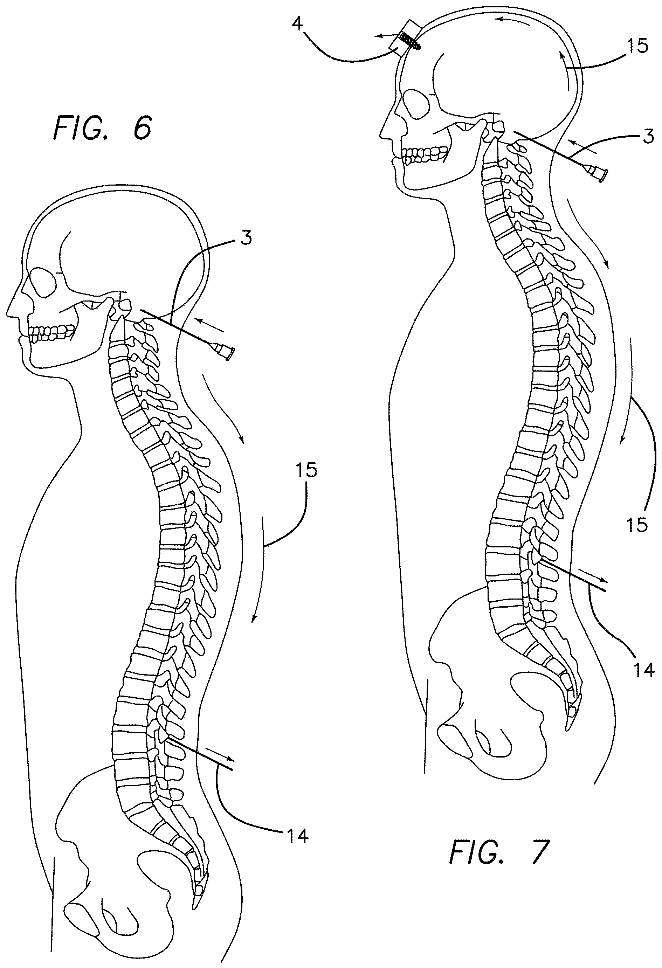

Fig. 6 is a simplified side cross sectional diagram of a human body showing an approach to cool the spinal cord by inserting needles into the cisterna magna and into the subarachnoid space of the spinal canal *through* a lumbar puncture. By circulating cooled aCSF into the cisternal magna and directing it out the lumbar puncture, a pump/cooling system will direct flow of aCSF down the spinal canal. Depending on the situation, cooled aCSF can be directed into the lumbar puncture needle and exit from the cisterna magna needle.
Fig. 7 is a simplified side cross sectional diagram of a human body illustrating a method to cool both the brain and the spinal cord by using the entry point of cooled aCSF in the cisterna magna and directing flow out both the exit port in the skull and the lumbar puncture below.
Fig. 8 is a simplified side cross sectional diagram of a human head illustrating the advantage of using convection cooling to more rapidly cool the brain when the patient is lying supine with cool CSF below and wanner CSF above resulting in an additional "motor" effect causing the brain to cool even faster.

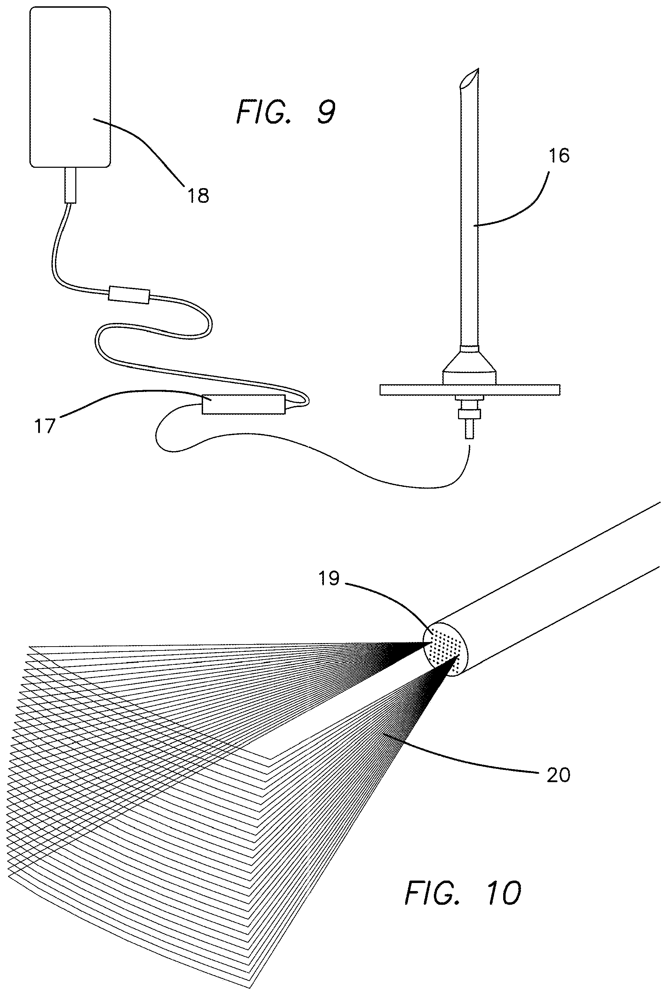

Fig. 9 is a diagram of the components of a phased array ultrasound device within a 2 mm diameter cannula (within a 17-gauge needle) comprised of the needle, an electronic interface unit, and a portable handheld monitor.
Fig. 10 is a diagram of the transmitting and receiving end of the ultrasound device of Fig. 9 illustrating the use of 64 or more elements in the phased array imaging, a field of view of up to 30 degrees or more, and penetration of up to 2.5 cm.

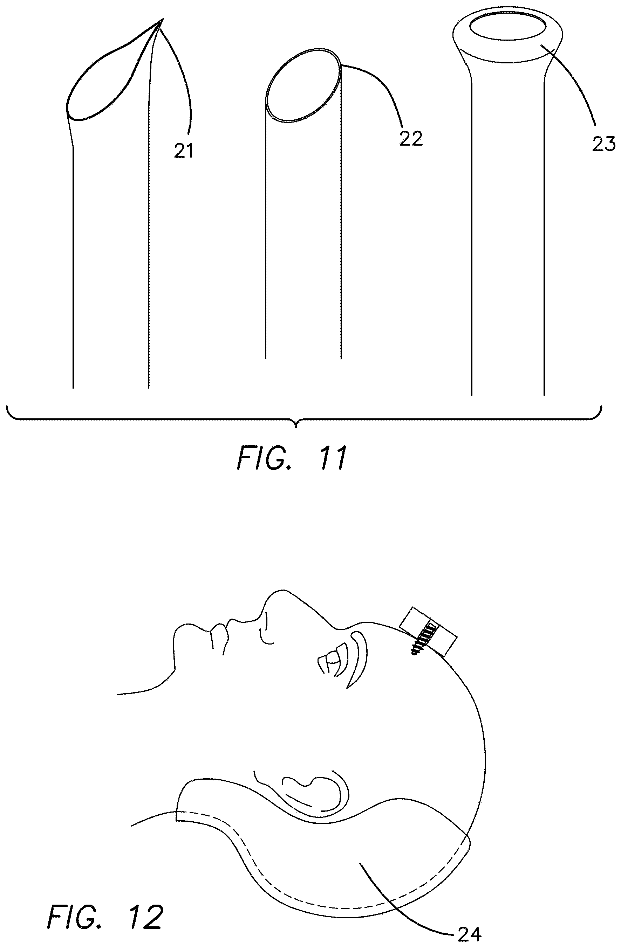

Fig. 11 is a perspective view of the terminal end of the ultrasound needle at different times, illustrating the concept of using temperature sensitive alloys such as Nitinol, After penetration by the needle into the cisterna magna, the cooled aCSF entering the needle will cause it to change its shape making the cutting edge dull with blunting of the pointed tip. In addition, the temperature sensitive alloy can reshape the tip to allow flow of aCSF to create less turbulence and direct streaming on neurological tissue.
Fig. 12 is a simplified side cross sectional diagram of a human head illustrating a "skull cap" which will fit like an exoskeleton to give fixation and stability to the ultrasound needle unit.

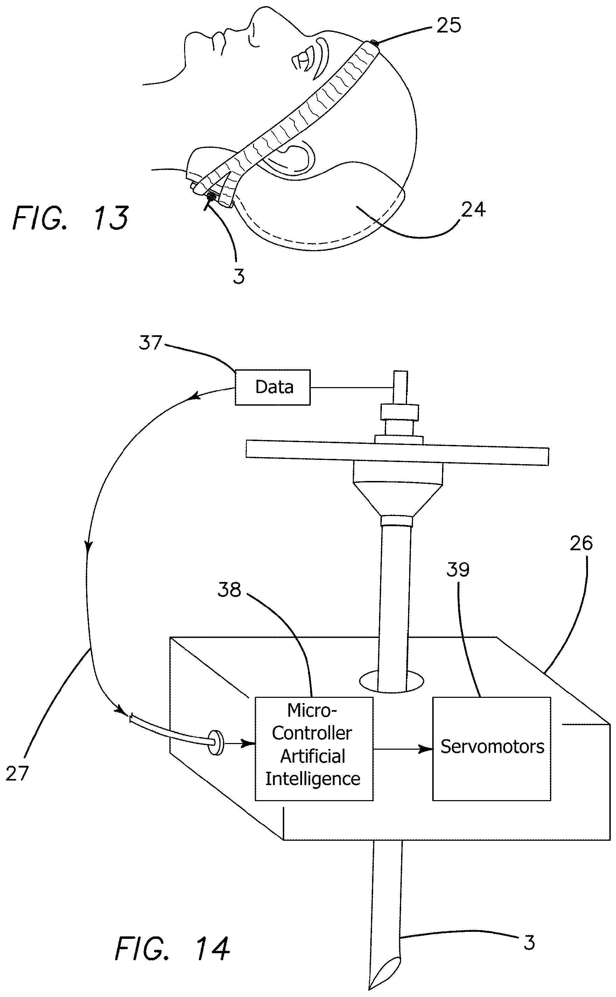

Fig. 13 is a simplified side cross sectional diagram of a human head illustrating the use of a band from the forehead to the upper cervical spine which provides a semi-rigid unit to give support and fixation to the needle. It will be sturdy enough to allow attachment to a servomotor system described in Fig. 14. The fixation anteriorly provided by the screw in the trephine is fixated in the frontal bone. The fixation point posteriorly acts a firmly fitting exoskeleton like a "skull cap" or similar device over the head and upper neck to give stability and precision to the ultrasound needle to enter the neck and into the cisterna magna.

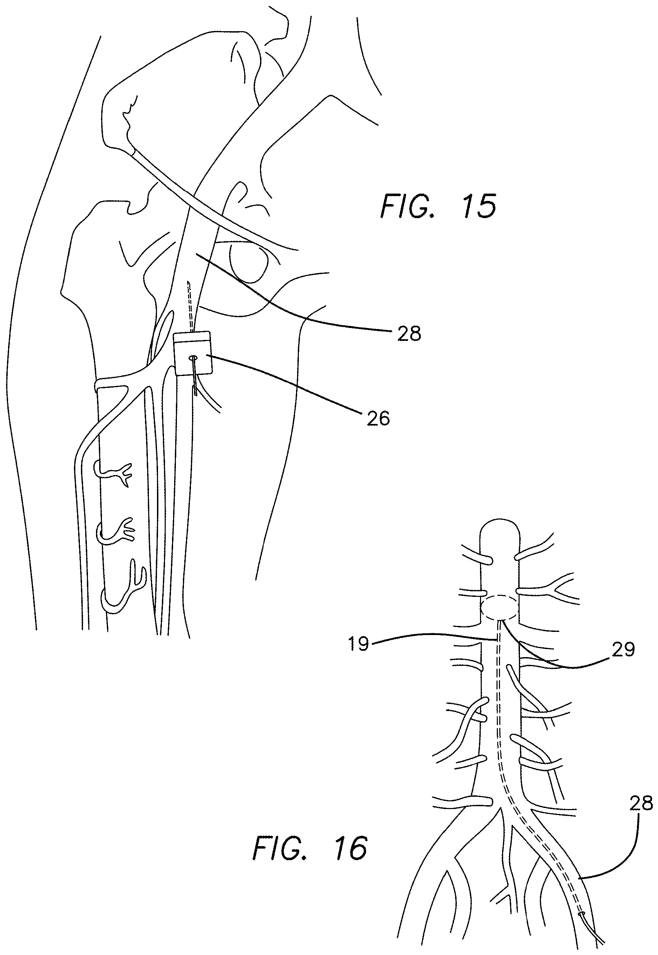

Fig. 14 is a diagram of a system that semi-autonomously or robotically can insert the phased array ultrasound needle into the cisterna magna or femoral artery described in Fig. 15. Once the needle is put into proper position, the information from the phased array ultrasound needle is sent to the microprocessor/artificial intelligence unit and then to the servomotors which control the insertion of the needle to the target site. This requires a fixation unit that is sturdy and stable as shown in Fig. 13.
Fig. 15 is a simplified front cross sectional diagram of the femoral artery target site and the associated anatomy for localization of the needle entry.
Fig. 16 is a diagram of the aorta showing the trajectory of a phased array ultrasound cannula as it enters the aorta from the femoral artery. With a 30 degrees range of view and a penetration of up to 2.5 cm, a balloon tamponade of the aorta is made more precise and performed quickly.

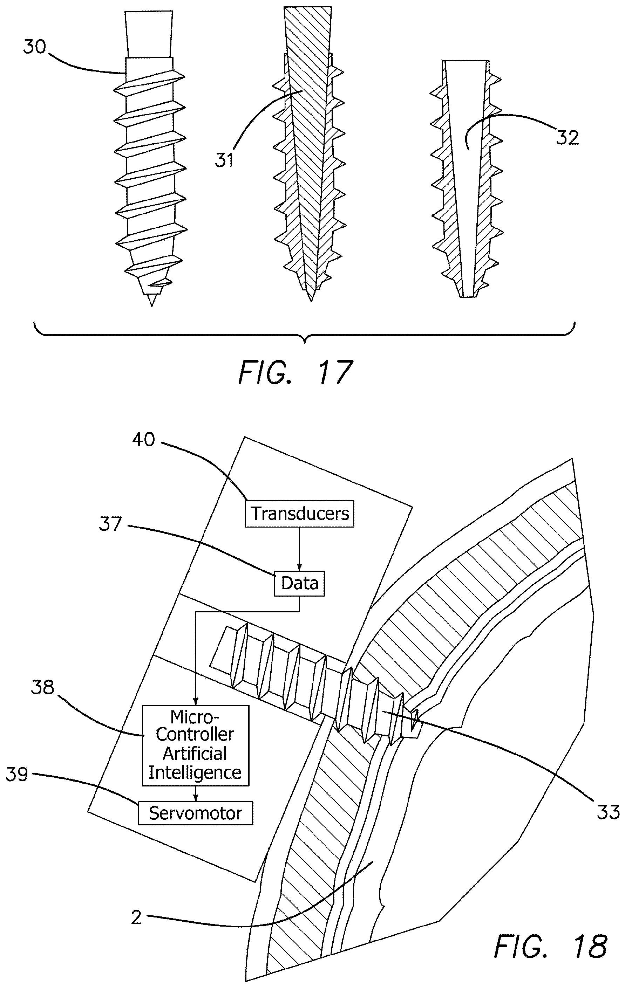

Fig. 17 is a series of side plan view, and two side cross sectional views of a hollow trephine screw with a central trocar, which when removed will leave a cone-shaped tunnel for exact placement of the ultrasound probe into the subarachnoid space safely, precisely, and with good fixation. Once in place, this trephine screw allows for removal of cerebrospinal fluid from the subarachnoid space.
Fig. 18 is an enlarged view illustrating the trephine device unit that houses four or more transducers to give ultrasound information to the microcontroller that semi-autonomously controls the servomotor to direct the screw *through* the skull to fixate it firmly to the bone and to stop it once it enters the subarachnoid space.

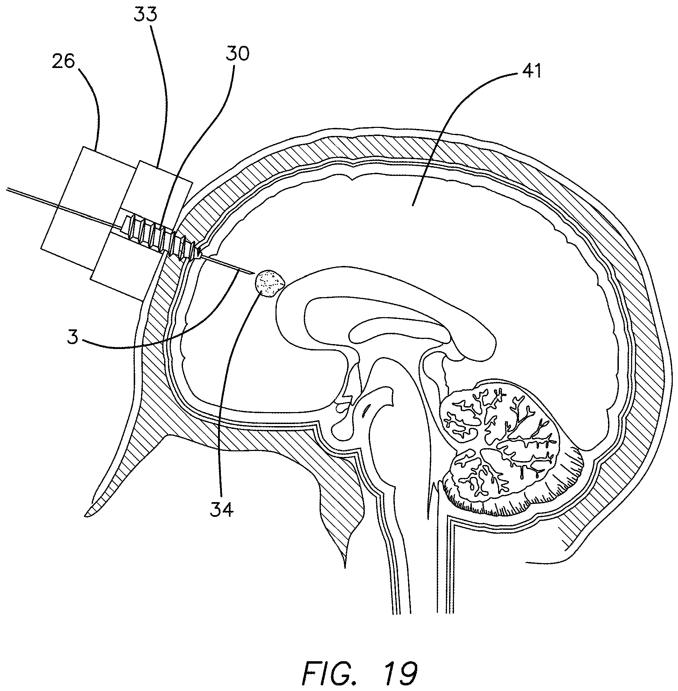

Fig. 19 is a simplified side cross sectional diagram of a human head which illustrate the device of Fig. 10, namely an ultrasound needle including a 2 mm inside diameter needle with 64 or more elements which can characterize the tissue characteristics of a tumor in contrast to normal tissue. The instrumented ultrasound needle gives information to image, to direct the semi-autonomous or robotic insertion of the needle, and to treat a glioblastoma tumor. The ultrasound transducers generate energy at the tip of the needle in proximity of the tumor. After pretreatment of the tumor, the same needle allows for the administration of microliters of various treatment modalities. Following administration of medication, the ultrasound needle tip images the tissue to determine the location of the treatment effect in relation to normal brain tissue.

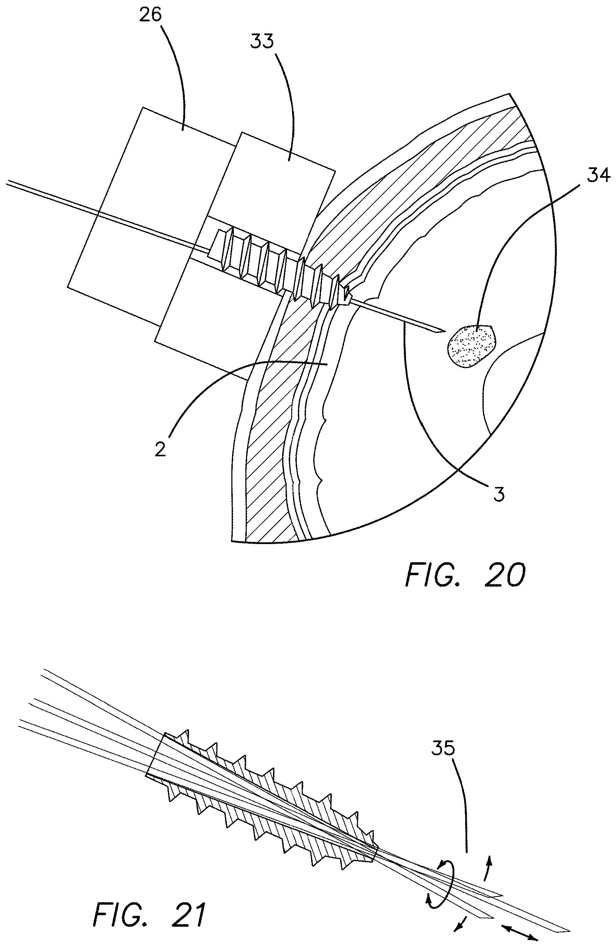

Fig. 20 is a side cross sectional view of an insertion site in the human skull illustrating a combination of the devices of Figs. 14 and 18 which includes the trephine unit with the ultrasound needle unit to carefully direct the needle into the subarachnoid space and fixate it in position for tissue diagnosis, to map the brain tissue, and to develop a trajectory of the ultrasound needle into the brain.
Fig. 21 is a side cross sectional diagram of an ultrasound needle having a diameter of 2 mm and fitting within a 3 to 4 mm hollow cone space within the surrounding screw, providing sufficient freedom to precisely place the ultrasound needle using the semi-autonomous or robotic system with high precision.

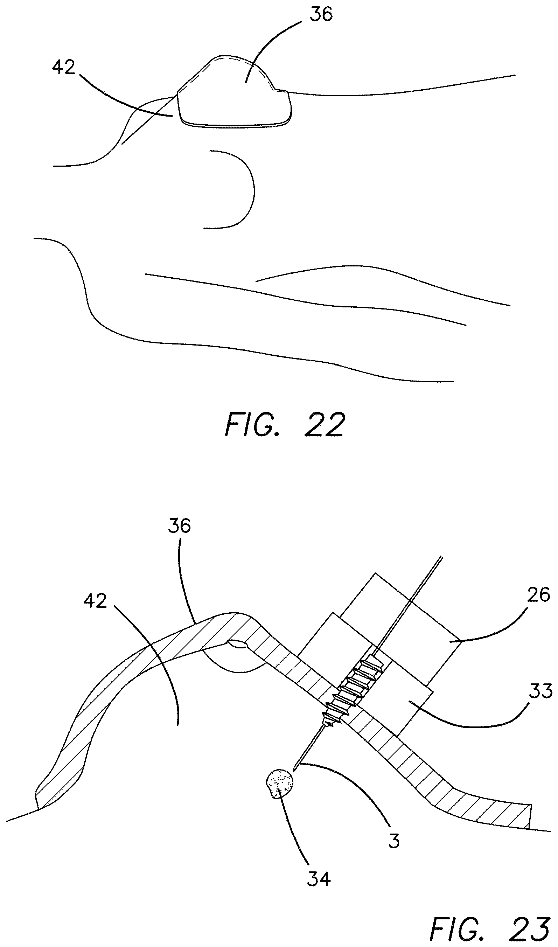

Fig. 22 is a diagram of a perspective view of a human body illustrating how an exoskeleton can be used to treat solid tumors elsewhere in the body. From laser scanning data or from other imaging data such as MRI or CT scanning, a 3-dimensional printing of an exoskeleton can be expeditiously produced to fit firmly and precisely with minimal movement. This will give solid infrastructure for fixation of the trephine and/or the ultrasound needle unit to precisely enter the body and target a tumor to within 1 mm proximity to the tumor to diagnose and treat with ultrasound energy and to administer therapeutic medication to the tumor with great precision and monitoring of the effect on the tumor and the surrounding normal tissue.
Fig. 23 is a diagram of a side cross sectional view of the use of the exoskeleton on a human breast to give stability and precision to the semi-autonomous or robotic unit to direct the ultrasound needle to within 1 mm of the tumor of the breast, where it can diagnose, delineate, and treat the tumor with minimal injury to the normal tissue.

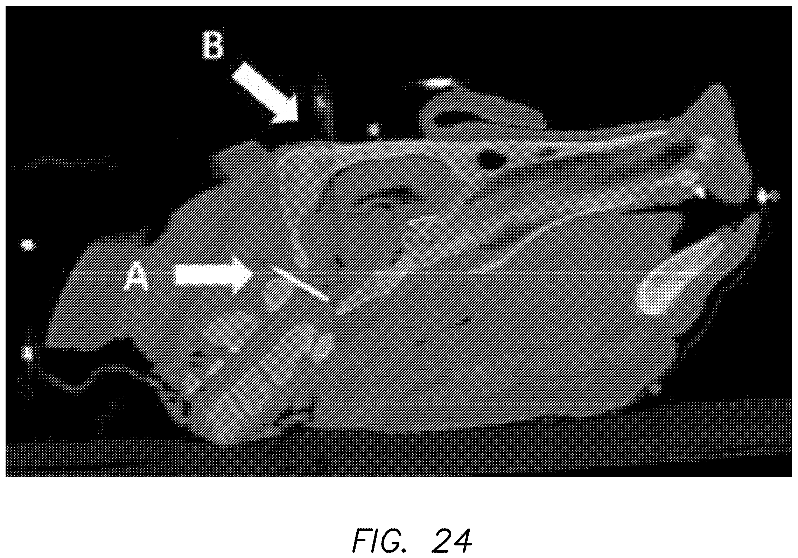

Fig. 24 is a sagittal view of the decapitated head and neck of a pig used in an animal study. This CT scan confirms the location of the cisterna magna needle and the location of the thermistors denoted by the while arrows.

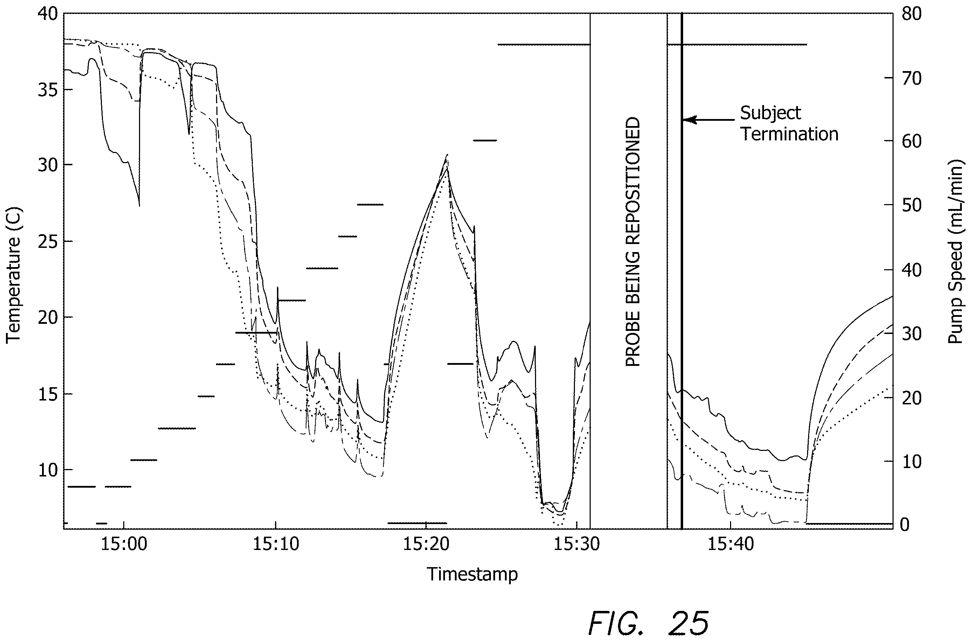

Fig. 25 is a graph of the measured temperature verses time in the porcine experiment depicted in Fig. 24.

The disclosure and its various embodiments can now be better understood by turning to the following detailed description of the preferred embodiments which are presented as illustrated examples of the embodiments defined in the claims. It is expressly understood that the embodiments as defined by the claims may be broader than the illustrated embodiments described below.

### Detailed Description of the Preferred Embodiments

### Animal Studies:

The cisterna magna, located at the base of the brain, was chosen to be the entry point for the cooling devices due to ineffective previous attempts to cool the brain via a lumbar puncture. Entering through the spinal canal resulted in a rise in temperature of the aCSF due to the warmth of the circulating vessels in the spinal canal. By entering the largest cistern, the cisterna magna, a 17-gauge needle can be placed into the subarachnoid space and used to circulate cooled saline out through a vent in the forehead. Using this methodology, initial work on more than 50 recently deceased pigs provided promising results and encouraged testing in vivo.

To test in vivo, our approach was combined with thermistors, placed at various depths (5 mm increments) in the brain to monitor the temperature of the tissue and therefore the effects of the circulating cooled aCSF as depicted in the CT scan in Fig. 24. Brain temperature collected during the in vivo experiment demonstrated that cooling the brain can be accomplished within a large animal model under general anesthesia with warm blood flowing into the brain. Furthermore, once the specimen was terminated with cuthasol at 15:35, the brain temperature was monitored with no blood flow. The graph of Fig. 25 demonstrates that termination of inflow of warm blood from the heart results in additional cooling of the brain. These results are consistent with our hypothesis that it is imperative to consider the warm gradient of incoming blood when attempting to cool the brain with cooled aCSF. Fig. 25 is a graph of data collected from four implanted thermistors monitoring the brain temperature of a porcine model under general anesthesia and our proposed methodology of inducing hypothermia. Each thermistor is placed within the cortex at four different depths of the brain from the surface at 5 mm increments. The horizontal bars indicate the pump speed in mL/min. Within the first 10 minutes, the temperature drops to 15°C. After terminating the specimen at 15:35, the temperature continued to decrease until the experiment was concluded.

Contrarily, this radical system is designed for extreme cases of emergency, specifically targeting patients who are experiencing shock or have little to no blood flow to the brain. These cases would be more advantageous as there would be minimal heat from the circulating blood making cooling the brain even easier and therefore maximize the time for intervention.

Results from our in vivo studies give support to our approach of cooling the brain by cooling the CSF directly and allow convection cooling to increase the cooling effect in the subarachnoid space adjacent to the cortex where the neurons are located. Moreover, since the CSF flow from the cisterna magna starts at the base of the brain, cooling of the memory circuitry is cooled early. Since the vertebrobasilar circulation is adjacent to the cisterna magna, there will be cooling of the circulating blood without cannulating any blood vessel, resulting in additional cooling of deep structures.

We have demonstrated in our live animal studies the efficacy of the cooling system as shown in Fig. 1. The most important caveat is that once the needle is inserted, it must be immobilized immediately in order to prevent repositioning of the ultrasound needle 3 outside of the cisterna magna 1. The aCSF is refrigerated in the cooler 7 to a temperature in the range of 1 to 10°C, carried by the pump 8, through a filter 9 and into the needle 3 in the cisterna magna 1. The flow of cooled aCSF throughout the subarachnoid space 2 is very effective in cooling the brain at several levels, especially at the cortical surface where the more vulnerable neurons of the gray matter reside. In older adult patients, the subarachnoid space 2 is more capacious for there is natural shrinkage of the brain with age, resulting in more rapid exposure of the brain to the cooling effect with a larger volume of aCSF in circulation. The cooled aCSF in the cisterna magna 1 is near the basilar artery and the circulation of the Circle of Willis, which will have an additional cooling effect upon central structures, especially to areas of the brain that subserve memory. Note that the cooling system may initially be chemical cooling, which is a more simplified and effective method in emergency situations. Moreover, if the treatment is not necessarily to cool the brain but to change the temperature to a wanner state, a cooler and/or a warmer may be utilized in a similar system to result in the proper temperature for the irrigant. Thermistors are provided to monitor temperature within the subarachnoid space 2 at the exit of aCSF 5 in the frontal skull through the trephine 4 and at the entrance into the cisterna magna 1. An intracranial pressure manometer monitors intracranial pressure.

The disclosed apparatus and method provide a successful and safe way to rapidly cool the brain to prevent brain death by using deep hypothermia. A needle 3 is placed into the cisterna magna 1 safely, rapidly, and accurately to circulate cold artificial cerebrospinal fluid (or other isotonic solution) into the intracranial and subarachnoid space 2. By lowering the temperature of the artificial cerebrospinal fluid (aCSF) and circulating it around the blood vessels of the basal cisterns, the circulation of the brain will be cooled. Once the cooled aCSF reaches the subarachnoid space 2 especially in the back of the brain by a computer-controlled peristaltic pump, convection cooling will begin, for an exit will be made through the frontal bone for egress of the warmer fluid. The exiting aCSF will then be sterilely collected as illustrated in Fig. 1.

Alternatively, it will be cooled and filtered outside the body and recirculated as shown in Fig. 2 back into the inserted needle 3 which will be securely placed into the cisterna magna 1, In Fig. 2 recycling of aCSF 10 from the exit port in the skull to a pump requires a filtering system 9 to remove infectious agents and debris from the aCSF before it reenters the cisterna magna 1. Recycling aCSF 10 in a sterile environment has many advantages: it is easier to maintain a sterile environment; cooling is more efficacious; less aCSF is necessary to carry in an emergency; efficiency of space and of supplies may result. The inserted needle 3 will be fixated to a stable structure as described in Figs. 12 and 13 of the head and neck to prevent movement of the needle tip within the cisterna magna 1.

Fig. 12 illustrates one configuration of a method to fixate the needle tip after entry into the cisterna magna 1 including the use of an exoskeleton 24 placed over the posterior head and neck to give fixation, stability, and precision to the proper insertion of the ultrasound needle into the cisterna magna 1. The mastoid bones and/or the external auditory canals will be the posterior points of fixation while the trephine screw in the frontal bone will be the anterior point of fixation. A "skull cap" will fit like an exoskeleton 24 to give fixation and stability to the ultrasound needle.

Fig. 13 represents a second embodiment to fixate the needle after entry into the cisterna magna 1. It includes the use of a firm band placed around the forehead at the trephine which is firmly screwed into the frontal bone 25 and going around the upper cervical spine without causing compression injury to the ears. The external auditory canals will serve as reference points for locating the band in the proper plane. It gives stable fixation to the needle as it enters the cisterna magna 1. Moreover, if a servomotor system is used to semi-autonomously or robotically direct and move the needle into the cisterna magna 1, it will require a stable and fixed base from which it will direct the needle such as the "skull cap" exoskeleton 24 as described in Fig. 12.

This circulation of cooled aCSF in the subarachnoid space rapidly cools the entire brain including the structures at the base of the brain as well as the grey matter on the outer surface of the brain.

In Fig. 3 a single opening in the skull is planned in order to exit circulating aCSF from the subarachnoid space 2, and if the patient is lying supine it is best to place that opening toward the frontal lobe in order to take advantage of convection cooling as shown in Fig. 8. A semi-autonomous or robotic trephine 4 with a hollow screw serves to evacuate the fluid in Fig. 18. Through the same opening a temperature sensor 11 gives feedback about depth of cooling, efficacy of cooling, and rate of cooling. A pressure sensor (not shown) will also be necessary to prevent brain damage with too high a pressure during administration of aCSF. Knowing the temperature of fluid entering the cisterna magna 1 and leaving the exit point 4 as well as flow rate will give rate of cooling as well as efficacy of cooling. Fig. 8 explains the additive effect of convection cooling 13 to the pumping action on aCSF after it enters the cisterna magna 1 and settles in the occipital region of the head with the patient lying supine. With cool CSF in the occipital region below and relatively warmer CSF toward the frontal lobe above, convection cooling will occur with the warmer fluid being evacuated to drainage.

Thus, the disclosed apparatus and method successfully produce deep hypothermia of the brain safely and rapidly. In addition, this method will also be effective in cooling the spinal cord as described in Fig. 6 rapidly and safely to prevent swelling of the spinal cord after trauma or to protect the cord against ischemic injury; redirecting the flow of cooled aCSF from the cisterna magna 1 down to the lumbar area by venting through a lumbar puncture needle 14 results in cooling of the spinal cord,

Cardiac arrest, stroke, and exsanguination can result in brain death in five minutes unless the brain can be placed into suspended animation rapidly, giving the treating physician up to one hour or longer to save the life of the patient, which is provided by the apparatus and method of the disclosure.

The disclosed apparatus includes an ultrasound guided needle 3 as described in Figs. 9 and 10 with multiple elements configured to give a 3-dimensional ultrasound image in front of the entering needle. The image and ultrasound feedback are displayed on a video monitor similar in size to a cell phone, making it possible for a paramedic to enter the cisterna magna 1 of the brain rapidly and safely. In addition, the tip of needle 3 includes a temperature sensitive alloy as shown in Fig. 11 to reshape it to prevent cutting brain tissue and to facilitate gentle flow of aCSF through the needle 3. A similarly designed needle 3 facilitates entry semi-autonomously into arteries and other vessels in the body, such as the femoral artery when rapid and safe entry is needed in an emergency.

To rapidly cool the brain to prevent brain death by using deep hypothermia, it is crucial that a needle 3 be designed to be placed into the cisterna magna 1 safely, rapidly, and accurately to circulate cooled artificial cerebrospinal fluid or other isotonic solution into the intracranial and subarachnoid space 2.

Fig. 5 illustrates a new approach from the lateral side of the neck just below the mastoid bone 12. Gong et al. (J Neurosurg 129: 146-152, 2018) describe a Lateral Atlanto-occipital Space Puncture on 1, 008 punctures in which 98.3% were successful. This has the advantages of rapid insertion, proximity to the mastoid bone for fixation, and high success rate of insertion. Currently, this procedure is performed by neurological surgeons and neuro-radiologists using fluoroscopy which is not always available in the field, the emergency room, or the operating room. Moreover, mistakes can occur under the stress of emergency situations, and the sharp needle tip poses a hazard to the brain while in the cisterna magna for a prolonged period. Therefore, there is obvious need for new technology to enter the cisterna magna 1. This is addressed in this disclosure.

The lateral neck below the mastoid bone may be easier for the paramedic or nurse to locate and access. It is easily identified as the bony prominence behind the car as shown in Fig.5, it usually does not have to be shaved of hair, it is a stationary point of fixation, and immobilization of the needle 3 can be done quickly and easily. Insertion of the ultrasound guided needle 3 gives directional images on the handheld unit like a cellphone with visual and audio information displayed in real time as shown in Fig.9. Semi-autonomous or robotic insertion is also described. The data obtained from the tip of the ultrasound needle 3 directs the tip of the unit to its trajectory.

### Spinal Cooling

Fig. 6 shows a new approach to treating and/or preserving the spinal cord when subjected to direct trauma, blunt trauma, concussive injuries, ischemic trauma, explosive exposure, and surgical intervention that may jeopardize the circulation to the spinal cord. Sending cooled aCSF into the cisterna magna 1 and directing the flow 15 of aCSF down the spinal canal results in venting through a lumbar puncture needle 14. Similar to cooling the brain, cooling the spinal cord can be done by pumping aCSF into the cisterna magna 1 and venting to a drainage bottle. Recycling the aCSF from the lumbar puncture needle 14 back to the cisterna magna 1 by using a pump, filter, and cooler in series emulates the technique for brain cooling. Depending on fluid dynamics and location on the spinal cord that needs to be cooled, cooling can be initiated through the lumbar puncture needle 14 and vented above through the cisterna magna 1.

Fig. 7 relates to situations when both the brain and spinal cord needs to be cooled simultaneously. Such cases may include extensive dissecting of nortic aneurysms, explosive and concussive trauma, and other exposure to trauma such as radiation exposure. By cooling from the cisterna magna 1, both the brain and spinal cord can be cooled at the same time by draining cooled aCSF from vents in the head and the lumbar puncture sites.

### Prevention of the Sharp Needle Tip from Cutting the Brain

To prevent the sharp tip of the needle 3 from puncturing or lacerating the cisterna magna 1 or other neurological tissues in the brainstem, spinal cord, and brain, the tip of needle 3 is made of a shape-memory alloy that changes it shape when the temperature changes to a predetermined range as shown in Fig. 11. In addition to removing the sharpness of the needle tip, the outlet can be reshaped to maximize the outflow of the cooled aCSF while ameliorating flow patterns that may injure the adjoining neurological tissue. Nitinol is one shape-shifting alloy that was developed by the U.S. Navy and stands for nickel titanium alloy, and it has thermal memory.

Fig. 11 illustrates the concept of using temperature sensitive alloys such as Nitinol at the cutting tip of the needle. After penetration of the sharp pointed needle 21 into the cisterna magna 1, the shaft of the needle must be securely fixated at that determined location. The cannula is removed and cooled aCSF is administered through the needle shaft, causing the cutting tip to reshape into a blunted tip 22 with no sharp edges. If the pump is raised to a higher volume given rapidly, it is important to design a new shape for the end of the needle shaft to create gentle dispersion of the aCSF as it enters the cisterna magna 1. It may require a flared tip 23 or other shape as determined by computer simulation and modeling.

In the case of using a similarly designed needle 3 for rapid entry into a large vessel such as the femoral artery, the phased array cannula 19 within the tip of needle 3 delineates the various tissues until the needle 3 enters the artery, while the paramedic uses the handheld video and audio unit as depicted in Fig. 9 to rapidly guide the tip and the needle 3 into the vessel. Fig. 15 illustrates the femoral artery 28 target site and the various tissues and vessels that may cause confusion on insertion. The semi-autonomous unit 26 is fixated to anatomic structures that will give stability and fixation for insertion of the needle into the femoral artery 28. If there is confusion regarding differentiating the artery from the vein, a Doppler signal tells the difference. Once the needle tip 3 is in the artery, if it needs to be there for any extended time, the temperature-sensitive alloy tip 3 changes shape at a higher temperature of arterial blood to blunt any sharp surfaces to prevent damage to the artery as suggested by Fig. 11. The phased array cannula 16 is extended up the aorta to identify the circulation of the surrounding abdominal tissue as well as the vessels to structures such as the kidney, spleen, pancreas, liver, heart, and other organs as depicted by Fig. 16. The phased array elements have a field of view of 30 degrees, can penetrate forward for up to 2.5 cm, and have real time 3-D imaging as shown in Fig. 10. Fig. 16 shows the trajectory of a phased array ultrasound cannula as it enters the aorta from the femoral artery 28. The cannula has a 30 degree of range of view as well as penetration up to 2.5 cm, giving it a 3-dimensional real-time image of the vascular branches off the aorta. If there is life threatening exsanguination, balloon tamponade 29 of the aorta will be more precise and more quickly performed.

Fig. 10 demonstrates the effective 3-dimensional space in front of the 64-or-greater elements within a 2 mm inside diameter cannula configured to do phase array imaging. Ultrasound data can cover a field of view of 30 degrees and a depth of penetration of up to 2.5 cm 20. The use of phased array using multiple ultrasound elements is utilized throughout the medical industry. Volcano Corporation Systems images with a catheter ultrasound system has the Angio+^{™} Quantitative Coronary Analysis which automatically calculates lumen dimensions and stenosis in real time. Interson Corporation has commercially produced a small transducer system with commercially available hardware, using its own built-in electronics to apply to cardiac disease.

### Motorized Insertion of the Needle

Semi-autonomous or robotic technology has been in general practice for decades and is used in the navigation of self-driving cars, etc. Based on radar, camera data, and GPS information the "self-driving" car uses artificial intelligence to program the car to maneuver its way from home to work safely. Similarly, we utilize 3-dimensional ultrasound information from the tip of the needle to use artificial intelligence to program the servomotors to direct the needle to safely travel through the skin, fat, muscles, tendons, and blood vessels on its way to the cisterna magna. Once in the cisterna magna, the needle automatically locks in place to prevent damage to neurological tissue. This requires expert software development all of which has been done in other industries. The novelty of this invention is the application of a custom developed software to enable semi-autonomous or robotic insertion of the needle with several orders of magnitude greater precision than a self-driving car, in the order of a fraction of an mm within the target site.

As disclosed in Mathiassen et.al. "Visual Servoing of a Medical Ultrasound Probe for Needle Insertion, "2016 IEEE International Conference on Robotics and Automation (16-21 May 2016), percutaneous needle insertion guided by ultrasound imaging is routinely performed in hospitals. Automating these procedures increases placement accuracy and lowers time usage of health care personnel to perform these procedures. An important step in the automation is the estimation of the needle orientation and position in the ultrasound image. One approach to estimate the needle orientation and position is to have the needle aligned with the image plane of the ultrasound probe. Aligning the needle with the plane is difficult, even with accurate measurements and calibration of both needle and probe. Visual servoing to move the ultrasound probe is performed using a robot to align the image plane of the probe with the needle, which solves the problem of needle alignment. The method segments the needle and updates a set of visual features based on a model of the needle. A state machine is used to keep track of the alignment process, and different visual features are used to control the probe in the different states.

The methods algorithms, and apparatuses of using images collected from cameras to guide the steering, braking, and accelerating a vehicle exist in the field. For example, see "Autonomous Driving Control Device," U.S. Pat. Appl. 15/413568 and "Control Arrangement Arranged To Control An Autonomous Vehicle, Autonomous Drive Arrangement, Vehicle And Method" US Patent 9566983. In our technology, instead of using images from cameras we use images from our ultrasound probe. Instead of controlling the movement of a car, we control the movement of the probe. Overall, the concept and approach are similar. The algorithms will be different to accommodate the use of a different kind of image and the control of a different kind of actuation mechanisms. The means to make these adjustments are well within the ordinary skill in the art. There have been numerous patents issued on using ultrasound images as feedbacks to control a medical device. In particular, "Feedback in Medical Ultrasound Imaging for High Intensity Focused Ultrasound" US Patent 8343050, describes the use of ultrasound imaging to detect and monitor the small change in tumorous tissues as a result of applying high-intensity focused ultrasound (HIFU) to the tissues. The image is used as a feedback to control the focal point, intensity, and duration of the HIFU. In our case, we are using the same ultrasound imaging technique as a feedback to control the movement of the probe. The algorithm is adapted to control the servo motor instead of the HIFU. The basic principles and approach are the same.

A motorized inserter for the needle 3 to enter the cisterna magna 1 requires a micro-controller 38 with artificial intelligence to control a servomotor 39. Servomotor 39 is a rotary actuator or linear actuator that allows for precise control of angular or linear position, velocity, and acceleration. It is comprised of a suitable motor coupled to a sensor for position feedback as illustrated in Fig. 14. Fig. 14 depicts a system 2.6 that semi-autonomously or robotically inserts the needle 3 which is equipped with a phased array ultrasound cannula with an ultrasound data feedback unit 37 to the micro-controller 38 and micro-controller directed servomotor 39. Once the medical specialist sets the trajectory for the needle 3 on its way to the target, the cisterna magna 1, the ultrasound data feedback unit 37 directs the servomotor 39 in real time to insert needle 3. It is firmly attached to a fixation device on a site such as a "skull cap" in the shape of an exoskeleton 24 or similar device as described in Figs. 12 and 13 and which depends on information from the ultrasound data feedback unit 37 obtained from the phased array ultrasound system 16 as depicted in Figs. 9 and 10, Rapid, accurate, and safe insertion of the needle 3 into the cisterna magna 1 in an emergency will be facilitated by servomotor inserter 26. Fig. 9 shows the device used to direct the needle through the skin to the cisterna magna 1 by using known technology in a unique adaptation at the tip of a needle. By using a 2 mm diameter cannula within a cutting needle (17-gauge needle) 16, 64 or more ultrasound elements 19 shown in Fig. 10 are used to create a phased array ultrasound imaging field 20. By using raster graphics, a three-dimensional image is presented on a handheld portable monitor 18 in real time, An interface unit 17 converts the ultrasound data into imaging information to be displayed. Both visual and auditory feedback provided by monitor 18 aid in the insertion of the needle into the cisterna magna 1. Moreover, the shape of the cisterna magna 1 can be delineated for greater ease and accuracy of insertion. Once the needle 3 is fixated to a stationary site as described in greater detail below in relation to Figs. 12 and 13, the cannula 16 is removed from the cisterna magna 1 to allow for administration of cooled aCSF. In the situation of insertion of the phased array needle into the femoral artery, the cannula 16 can be directed into the aorta for visualizing the many vascular branches in the aorta.

Fig. . 22 shows how an exoskeleton 36 can be used to treat solid tumors elsewhere in the body, such as in the breast. From laser scanning data or from other imaging data such as MRI or CT scanning, 3-dimensional printing of a rigid exoskeleton can be expeditiously produced to fit the entry or operating site firmly and precisely with minimal movement. This will give solid structure for fixation of the trephine unit 33 and/or the ultrasound needle 3 to precisely enter the body and target tumor 34 to within 1 mm proximity of the tumor 34 to diagnose and treat with ultrasound energy and to administer therapeutic medication to the tumor 34 with precision and monitoring of its effect on the tumor and the surrounding normal tissue.

Fig. 23 shows the use of the exoskeleton 36 of Fig. 22 as tightly fixed to the breast to give stability and precision. The ultrasound needle 3 is then accurately directed to the tumor 34 within 1 mm to diagnose the tissue and to give treatment to the tumor 34 precisely without damage to the surrounding normal tissue.

In the case of insertion of a needle 3 into the femoral artery, a phased array ultrasound system will give anatomic information to facilitate the identification of the femoral artery in distinction to a femoral vein. Moreover, if the pulse of the femoral artery cannot be felt, then the anatomic information from the ultrasound system will better locate the femoral artery. A semi-autonomous ultrasound needle unit 14 to be inserted into the femoral artery also requires fixation of the unit as shown in Fig. 15, in this case to the inguinal ligament and/or bony protuberances in the configuration of an exoskeleton.

### Method and Device to Create a Safe and Rapid Insertion of a Trephine through the Skull:

In order to create an exit point in the forehead for cooled fluid to leave the subarachnoid space 2 during irrigation of aCSF to cool the brain, trephining of the bone in the skull must be accomplished safely, quickly, bloodlessly, and with precision by a single health care person. The unit must be self-contained including transducers and a semi-autonomous or robotic motorized insertion device as described in "Method of Insertion of the Needle" 26 in a "box" which will be attached to the area of the frontal bone. The "screw" 30 includes a hollow shaft within a 3 to 4 mm inside diameter screw with a trocar 31 which is carefully screwed into the cranial bone. Information received from the transducers within the device 26 control the semi-autonomous or robotic insertion of the screw 30 through the bone and safely into the subarachnoid space 2. Fig. 17 shows a hollow trephine screw 30 with a central trocar 31, which when removed leaves a cone-shaped tunnel 32 for exact placement into the subarachnoid space 2 safely, precisely, and with good fixation, Once in place, this trephine screw 30 allows for removal of cerebrospinal fluid from the subarachnoid space.

The insertion method for the semi-autonomous trephine unit includes the following: after choosing the point of entry, usually in the upper forehead near the hair line, an incision is made with a scalpel along the line of Kraissl in the natural skin fold from the skin down to the subcutaneous tissues overlying the frontal bone. Sterility is maintained, anesthesia with a vasoconstrictor is injected into the skin, and the length of the incision is between 1.0- and 1.5-mm. Insertion of self-retaining retractors are placed into the wound to give stability and hemostasis. The box unit 26 and the screw 30 are inserted into this opening and fixated to the retractors. The semi-autonomous or robotic insertion is activated and carefully directed through the bone into the subarachnoid space 2. The ultrasound data is obtained from four or more transducers 40 within the trephine "box" attached to the scalp. The micro-controller 38 with artificial intelligence directs the servomotor 39 which in turn screws the trephine into the skull. The final configuration is very stable and mobilized by the screw in the frontal bone as depicted in Fig.18.

The screw 30 in the frontal bone is rigidly attached, quite stable, and well fixated. It serves as one point upon which a headband is taken to the back of the head and attached to two or three points posteriorly to give greater stability during insertion of the ultrasound needle into the cistern magna as depicted in Fig. 13.

At the conclusion of the procedure, removal of the screw 30 can be done with local anesthesia, bone wax, if needed, placed into the small 3 to 4 mm opening, and a single suture placed across the skin. Since the incision is made in the line of Kraissl, there is minimal scarring, with the incision being in the natural skin fold.

In addition, for its central role in cooling the brain by creating an exit port, the disclosed method and device can be used in other brain operations including surgery for epidural hematoma, surgery for subdural hematoma, and for stereotactic intracranial surgery.

Hence an approach to the treatment of intracranial brain tumors, especially glioblastoma, is presented here combining the ultrasound needle 3 with the trephine 33. Fig. 18 shows the trephine device unit 33 that houses four or more transducers to give ultrasound infomtation to a semi-autonomous or robotic controlled servomotor to direct the screw through the skull to fixate it firmly to the bone and to stop it once it enters the subarachnoid space 2.

### A Novel Approach to the Diagnosis, Localization, and Management of Glioblastoma:

Glioblastoma, the most common intrinsic brain cancer, defies early diagnosis and treatment. From diagnosis with conventional imaging and brain biopsy to inevitable death of the patient within 4 to 16 months, these patients frequently undergo neurosurgery, radiation, and chemotherapy with little hope for a cure. We have the technology and expertise to accomplish characterization and diagnosis of disease, perhaps without taking a formal biopsy of the tissue by studying the tissue signature from data using ultrasonography. The disclosed approach is to do minimally invasive insertion of an ultrasound needle into the skull and into the subarachnoid space to scan the brain with a 30-degree field of view and a depth of up to 2.5 cm. Ultrasound data from the needle tip will image the tumor in 3-dimensions, and then be used to semi-autonomously or robotically insert the needle tip to within 1 mm of the target tissue. This requires precision and the development of servo-controlled devices to trephine 33 through the skull and to guide the ultrasound needle 3 to the target tissue.

The current state of the art for ultrasound imaging of the brain is to use transducers on the scalp and transmit energy through the bony skull. Low frequency transducers have the advantage in transmitting through the tissue of the scalp and bone, but resolution is low. In order to have high resolution imaging at the cellular and/or tissue level, the ultrasound transducer must be closer to the object and have high frequency imaging. This poses a dilemma. Therefore, the ideal ultrasound design would be to have a non-invasive device with penetration to the site of the disease with high frequency transducers. This can be done by making a trephine 33 opening through the bony skull sufficiently small to be minimally invasive, and using an ultrasound unit designed to be placed within the profile of a needle 3. Moreover, the ultrasound needle 3 is inserted into soft tissue of the body using semi-autonomous or robotic motorized systems with precision. Recent basic laboratory research by Sheehan et al. reported positive effects of use of ultrasound radiation to augment the effect of medications on the death of glioblastoma cells in culture (Kimball Sheehan et al. Investigation of tumoricidal effects of sonodynamic therapy in malignant glioblastoma brain tumors. J. Neuro-Oncology. 148, 9-16, 2020).

What is disclosed above is a use of an ultrasound needle 26 and semi-autonomous or robotic trephine 33 used beyond the initial purpose of cooling the brain in the case of brain death. The basis of this approach is sound, for it utilizes the elements in the tip of the needle 3 used for imaging, to be programmed to generate energy from the same transducers in the tip of the same needle 3. This method for the treatment of glioblastoma of the brain depends on an intimate relationship of the semi-autonomous or robotic ultrasound needle 26 and the semi-autonomous or robotic trephine 33 joining together to form a unique, precision, and stable platform. The design and the characteristics of the ultrasound needle 26 and the trephine 33 have been described above. The application of this technology creates a new approach to the diagnosis, localization, and management of glioblastoma of the brain.

Moreover, the ultrasound needle includes at least 64 elements within a 2 mm diameter cannula in the tip of the needle has a definition to less than 0.1 mm, with a field of view of 30°, and a depth of up to 2.5 cm. It is capable not only of imaging the shape and size of the tumor, but also is able to render specific tissue characteristics when ultrasound is passed near or within the tumor and through normal tissue. Therefore, tissue diagnosis may be done with ultrasound alone. After the semi-autonomous or robotic insertion of the hollow screw 30 is made down to the level of the subarachnoid space 2, the trocar 33 of the screw 30 is removed and replaced with the ultrasound needle 3 surrounded by its own semi-autonomous or robotic unit 26. These two units act as one and are securely attached, giving extreme accuracy and fixation while the needle 26 is sent deeper within the cranial cavity. Fig. 19 shows the use of the device of Fig. 10, namely an ultrasound needle 3 including a 2 mm inside diameter needle 3 with 64 or more ultrasound elements which characterize the tissue of a tumor in contrast to surrounding normal tissue. The ultrasound elements give information to image, to direct the semi-autonomous or robotic insertion apparatus 26 of the needle 3 through the semi-autonomous or robotic trephine 33, and to treat a glioblastoma tumor 34. The ultrasound transducers generate energy at the tip of the needle 3 in proximity of the tumor 34. After pretreatment of the tumor 34, the same needle 3 will allow for the administration of microliters of various treatment modalities. Following administration of medication, the ultrasound needle 3 can then image the tissue to determine the location of the treatment effect.

Ultrasound (US) guided biopsy is a medical procedure routinely performed in clinical practice. This task could be performed by robotic systems to improve the precision in the execution and then the safety for the patient. Both robotic and human procedures greatly benefit from real-time localization of the needle in US images. This information guides the robot or the specialists to the correct target point avoiding critical structures. In Mathiassen et.al. "Real Time Biopsy Needle Tip Estimation in 2D Ultrasound Images," 2013 IEEE International Conference on Robotics and Automation (6-10 May 2013) a needle localization method able to extract the needle orientation and the tip position in real time from B-mode US images is disclosed. The results show an improvement in term of localization accuracy compared to previous works in literature.

As disclosed in Mathiassen, "Robust Real-Time Needle Tracking in 2-D Ultrasound Images Using Statistical Filtering", IEEE Transactions on Control Systems Technology, 2017, 25 (3) 966-978, percutaneous image-guided tumor ablation is a minimally invasive surgical procedure for the treatment of malignant tumors using a needle-shaped ablation probe. Automating the insertion of a needle by using a robot increases the accuracy and decreases the execution time of the procedure. Extracting the needle tip position from the ultrasound (US) images verifies that the needle is not approaching any forbidden regions (e.g., major vessels and ribs), and also is used as a direct feedback signal to the robot inserting the needle. A method for estimating the needle tip has previously been developed combining a modified Hough transform, image filters, and machine learning. A method of introducing a dynamic selection of the region of interest in the US images and filtering the tracking results using either a Kalman filter or a particle filter is also known. The results show a significant improvement in precision and more than 85% reduction of 95th percentile of the error compared with the previous automatic approaches. The method runs in real time with a frame rate of 35.4 frames/s. The increased robustness and accuracy make the disclosed algorithm usable in autonomous or robotic surgical systems for needle insertion.

Fig. 20 is an enlargement of a portion of Fig. 19 showing the insertion site, and illustrates a combination of the devices of Figs. 17 and 19 including the trephine unit 33 with the ultrasound needle unit 26 to carefully direct the ultrasound needle 3 into the subarachnoid space 2 and fixate it in position for tissue diagnosis, mapping of the brain tissue, and developing a trajectory of the ultrasound needle 3 into the brain.

Fig. 21 shows how the design allows for precise directional guidance of the ultrasound needle 3 to its target at the glioblastoma tumor 34. Because the ultrasound needle 3 has a diameter of 2 mm and fits within a 3 to 4 mm hollow cone space within the surrounding stabilizing screw 30, precision placement of the ultrasound needle 3 using the semi-autonomous or robotic system 26 is performed with accuracy.

Since the hollow core within the screw 30 is shaped like a cone, there will be some play of the 2 mm diameter needle within the 3 to 4 mm hollow screw trephine. Therefore, control with the semi-autonomous or robotic motor allows the needle 3 to extend its range to cover a greater area than 30°. Imaging of the tissue immediately in front of the ultrasound needle to a depth of up to 2.5 cm will delineate the shape and size of the tumor. Moreover, because tissue density can be determined with ultrasound it will be possible to diagnose glioblastoma cells from normal tissue. To corroborate the ultrasound diagnosis, it is possible to do a needle biopsy through the needle 3 for confirmation.

If it is decided to direct the needle 3 deeper into the brain tissue with the semi-autonomous or robotic control and ultrasound information, it can be slowly and precisely placed up to the border of the tumor 34 or even within the tumor 34. The transducers can then be programmed to generate ultrasound energy in front of the needle tip 3 into the tumor 34. The addition of chemotherapeutic agents, immune therapy, or other modality can be given through the needle tip 3 in well-controlled small microliter volumes. The effect of the injection into the tissue can be ascertained by using ultrasound imaging to look for abnormal tissue response. An intracranial pressure gauge (not shown) shows if there is swelling of the tissue causing increased intracranial pressure. If need be, the hypothermia of the brain procedure can then be applied to cool the brain and prevent swelling.

### Semi-autonomous or robotic insertion of the ultrasound needle:

The semi-autonomous or robotic insertion of the needle into the cisterna magna is accomplished by using servo-controlled motors guided by information obtained from the 64 elements within the tip of the needle 3. The information will have a 3-dimensional space with a 30-degree range and a depth of up to 2.5 cm as shown in Fig. 10. Artificial intelligence with self-learning algorithms give machine learning to the micro-controller 38 in the device 26 in Fig. 14. Semi-autonomous or robotic insertion and servo-controlled motors enablement are described under "Motorized Insertion of the Needle."

### Semi-autonomous or robotic insertion of screw trephine:

The semi-autonomous or robotic insertion of the screw trephine 33 through the skull into the subarachnoid space will use a servo-controlled motor (not shown) guided by information obtained from four or more transducers in the unit 26 placed on the scalp. In Fig. 18, the information includes ultrasound data of the bone thickness and the interfaces of the dura and the arachnoid and the subarachnoid space 2 containing cerebrospinal fluid. Once the controller ascertains the proper interface in relationship to the hollow-bore screw 30 with the trocar 21, the fixation of the screw 30 within the bone will automatically be completed. Semi-autonomous or robotic insertion and servo-controlled motors enablement are described under "Motorized Insertion of the Needle."

## Claims

1. An apparatus comprising:
a needle having a tip for puncture through a patient's skin;
a phased array of ultrasound elements for generating an ultrasound image, the phased array being disposed in the tip of the needle; and
an ultrasound imaging system in a lightweight hand-held monitor with audio capabilities communicated to the phased array in the tip of the needle to generate an image of all tissues from a skin surface down to a target tissue within a field of view of at least 30 degrees or more with penetration of up to 2.5 cm or more,
where the tip of the needle has a sharp cutting shape for entry into a patient's cisterna magna and
where the tip of the needle is composed of a shape-shifting alloy to respond to a change in temperature when cooled artificial cerebrospinal fluid (aCSF) enters the needle resulting in a change of shape to a dull edge and blunt tip to avoid injuring brain tissue proximate to the cisterna magna.

2. The apparatus of claim 1 , where the resulting change of shape allows for a gentle dispersion of the aCSF instead of a forcefully directed flow of fluid which may injure the cisterna magna and the proximate brain tissue.

3. The apparatus of claim 1, further comprising a servo-controlled motorized inserter communicated with the ultrasound imaging system to guide the needle into the cisterna magna using the ultrasound image data from the phased array elements in the needle tip.

4. The apparatus of claim 1, further comprising a cannula disposed in the needle tip, where the phased array of ultrasound elements is disposed in the cannula, and further comprising a semi-autonomous or robotic unit communicated with the ultrasound imaging system, wherein the semi-autonomous or robotic unit is configured to direct the phased array cannula into arterial circulation of a patient's femoral artery for rapid and accurate placement of the needle into the femoral artery with the phased array cannula available to assist in selective balloon tamponade of aortic circulation or its branches.

## Patentansprüche

1. Einrichtung, umfassend:
eine Nadel, die eine Spitze zum Durchstechen einer Haut eines Patienten aufweist;
ein phasengesteuertes Array von Ultraschallelementen zum Erzeugen eines Ultraschallbildes, wobei das phasengesteuerte Array in der Spitze der Nadel angeordnet ist; und
ein Ultraschallbildgebungssystem in einem leichten Handmonitor mit Audiofunktionen, verbunden mit dem phasengesteuerten Array in der Spitze der Nadel, um ein Bild aller Gewebe von einer Hautoberfläche nach unten bis zu einem Zielgewebe innerhalb eines Sichtfelds von mindestens 30 Grad oder mehr mit einer Eindringung von bis zu 2,5 cm oder mehr zu erzeugen,
wobei die Spitze der Nadel eine scharfe Schneideform für einen Eintritt in eine Cisterna magna des Patienten aufweist und
wobei die Spitze der Nadel aus einer formveränderlichen Legierung, um auf eine Temperaturänderung zu reagieren, besteht, wenn gekühltes künstliches Zerebrospinalfluid (aCSF) in die Nadel eintritt, was in einer Formänderung hin zu einer stumpfen Kante und einer stumpfen Spitze resultiert, um ein Verletzen von Hirngewebe nahe der Cisterna magna zu vermeiden.

2. Einrichtung nach Anspruch 1, wobei die resultierende Formänderung eine sanfte Verteilung des aCSF anstelle eines kräftig gelenkten Fluidflusses, der die Cisterna magna und das nahe Hirngewebe verletzen könnte, ermöglicht.

3. Einrichtung nach Anspruch 1, ferner umfassend einen servogesteuerten motorisierten Einführer, verbunden mit dem Ultraschallbildgebungssystem, um die Nadel unter Verwendung der Ultraschallbilddaten von den phasengesteuerten Array-Elementen in der Nadelspitze in die Cisterna magna zu führen.

4. Einrichtung nach Anspruch 1, ferner umfassend eine Kanüle, die in der Nadelspitze angeordnet ist, wobei das phasengesteuerte Array von Ultraschallelementen in der Kanüle angeordnet ist, und ferner umfassend eine halbautonome oder robotische Einheit, verbunden mit dem Ultraschallbildgebungssystem, wobei die halbautonome oder robotische Einheit konfiguriert ist, um die phasengesteuerte Array-Kanüle in einen arteriellen Kreislauf einer Femoralarterie des Patienten zu lenken, zum schnellen und genauen Platzieren der Nadel in der Femoralarterie, wobei die phasengesteuerte Array-Kanüle verfügbar ist, um bei einer wahlweisen Ballontamponade von Aortenkreislauf oder seiner Abzweigungen zu helfen.

## Revendications

1. Appareil comprenant :
une aiguille munie d'une pointe permettant de percer la peau d'un patient ;
un réseau phasé d'éléments ultrasonores destiné à générer une image ultrasonore, le réseau phasé étant disposé dans la pointe de l'aiguille ; et
un système d'imagerie ultrasonore dans un moniteur portable léger avec des capacités audio communiquées au réseau phasé dans la pointe de l'aiguille pour générer une image de tous les tissus depuis la surface d'une peau jusqu'à un tissu cible dans un champ de vision d'au moins 30 degrés ou plus avec une pénétration allant jusqu'à 2,5 cm ou plus,
où la pointe de l'aiguille présente une forme de coupe tranchante pour pénétrer dans la citerne cérébello médullaire d'un patient et
où la pointe de l'aiguille est composée d'un alliage changeant de forme pour répondre à un changement de température lorsque du liquide céphalorachidien artificiel (aCSF) refroidi pénètre dans l'aiguille, ce qui entraîne un changement de forme vers un bord émoussé et une pointe arrondie pour éviter de blesser le tissu cérébral à proximité de la citerne cérébello médullaire.

2. Appareil selon la revendication 1, où le changement de forme qui en résulte permet une dispersion douce de l'aCSF au lieu d'un écoulement de fluide dirigé avec force qui pourrait blesser la citerne cérébello médullaire et le tissu cérébral proche.

3. Appareil selon la revendication 1 comprend en outre un applicateur motorisé à servocommande relié au système d'imagerie ultrasonore pour guider l'aiguille dans la citerne cérébello médullaire à l'aide des données d'image ultrasonore provenant des éléments à réseau phasé dans la pointe de l'aiguille.

4. Appareil selon la revendication 1, comprenant en outre une canule disposée dans la pointe de l'aiguille, où le réseau phasé d'éléments ultrasonores est disposé dans la canule, et comprenant en outre une unité semi-autonome ou robotique relié au système d'imagerie ultrasonore, dans lequel l'unité semi-autonome ou robotique est configurée pour diriger la canule à réseau phasé dans la circulation artérielle de l'artère fémorale d'un patient pour un placement rapide et précis de l'aiguille dans l'artère fémorale, la canule à réseau phasé étant disponible pour aider au tamponnement sélectif par ballonnet de la circulation aortique ou de ses branches.
